(19) Europäisches Patentamt / European Patent Office / Office européen des brevets

(11) **EP 3 367 986 B1**

## (12) EUROPEAN PATENT SPECIFICATION

(45) Date of publication and mention
of the grant of the patent:
**29.03.2023 Bulletin 2023/13**

(21) Application number: **16860863.6**

(22) Date of filing: **28.10.2016**

(51) International Patent Classification (IPC):
*A61F 13/00* (2006.01)    *A61F 13/02* (2006.01)
*A61M 1/00* (2006.01)    *A61M 35/00* (2006.01)
*A61N 1/04* (2006.01)    *A61N 1/36* (2006.01)
*A61N 5/06* (2006.01)

(52) Cooperative Patent Classification (CPC):
**A61F 13/00068; A61F 13/00; A61F 13/00063;
A61F 13/02; A61M 1/80; A61M 1/92; A61M 1/94;
A61M 1/962; A61M 1/985; A61M 35/30;
A61N 1/0492; A61N 1/36014;** A61M 1/964;
A61M 2205/0277; A61M 2205/051;    (Cont.)

(86) International application number:
**PCT/US2016/059291**

(87) International publication number:
**WO 2017/075331 (04.05.2017 Gazette 2017/18)**

## (54) TISSUE TREATMENT DEVICE AND METHOD

GEWEBEBEHANDLUNGSVORRICHTUNG UND -VERFAHREN

DISPOSITIF ET PROCÉDÉ DE TRAITEMENT DE TISSU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.10.2015 US 201562248422 P**

(43) Date of publication of application:
**05.09.2018 Bulletin 2018/36**

(73) Proprietor: **Advanced Dressing, LLC
Cleveland, OH 44114 (US)**

(72) Inventors:
• **DONDA, Russell, S.**
**Cleveland, OH 44114 (US)**
• **MANICKAM, Sundar**
**Cleveland, OH 44114 (US)**
• **MIDDAUGH, Richard, L.**
**Cleveland, OH 44114 (US)**
• **LASH, Thomas, E.**
**Cleveland, OH 44114 (US)**
• **WOJCIECHOWSKI, Timothy**
**Cleveland, OH 44114 (US)**

• **WOLTER, John, D.**
**Cleveland, OH 44114 (US)**
• **BUAN, John**
**Cleveland, OH 44114 (US)**

(74) Representative: **Ruttensperger Lachnit Trossin
Gomoll
Patent- und Rechtsanwälte
PartG mbB
Postfach 20 16 55
80016 München (DE)**

(56) References cited:
WO-A1-01/03619     WO-A1-2015/054040
US-A1- 2001 020 145   US-A1- 2005 070 835
US-A1- 2006 235 358   US-A1- 2008 183 119
US-A1- 2011 045 222   US-A1- 2014 109 890
US-A1- 2015 159 066   US-B1- 6 171 368
US-B1- 7 108 440    US-B1- 8 915 894

    **(Cont. next page)**

(52) Cooperative Patent Classification (CPC): (Cont.)
A61M 2205/054; A61M 2205/364; A61N 5/0616;
A61N 2005/0652

**Description**

BACKGROUND

The pores of the skin can become occluded with impurities. Negative pressure has been used to generate a partial vacuum to aid in removing impurities from the pores. Negative pressure is a term used to describe a pressure that is below normal atmospheric pressure. At room temperature and at sea level, a defined volume of air contains molecules moving in random directions, and these moving molecules exert a force that is equal to the normal atmospheric pressure of approximately 760 mmHg (about 1 bar). Negative pressure has been achieved by removing air from an area of interest, for example at a tissue site via a suction pump.

Devices for the generation of topical negative pressure at the surface of a person's skin have been used for many hundreds of years to treat humans. For example, the cupping technique, which relates to positioning a mouth of a rigid vessel containing hot air on a human's skin, is a well-known technique. Spring powered syringes and suction cups are other mechanical techniques that have been used for generating a partial vacuum on human tissue. In common with cupping, such other mechanical techniques have offered a limited topical negative pressure duration and little or no range of neutral to positive pressures. This is due to design constraints and that the cupping technique and other mechanical techniques are not self-contained and can hinder a user's mobility.

To enable a more prolonged application of topical negative pressure, powered systems, which include a vacuum generation source such as a pump, have been developed and many examples of such systems are used today for skin treatments and restorative purposes like the temporary removal of wrinkles. Many of these systems, however, are not convenient for users. Such known systems can be large, heavy, noisy, uncomfortable, and not simple for users to apply and initiate a controlled pressure condition. Such known systems also rely on an outside power or vacuum source to create topical negative pressure conditions.

From US 2005/0070835 A1 and from US 2008/0183119 A1 similar disposable wound-healing devices are known that incorporate a housing having a fluid-impermeable material having a cavity in the perimeter that can be sealed in an air-tight manner over a wound region of the patient. The device is capable of producing a negative pressure over the wound region by either removing oxygen from within the cavity, or absorbing fluid into the cavity and then removing the fluid from the cavity. The oxygen may be removed via chemical absorption, by an electrochemical cell or by a chemical reaction that cannibalizes oxygen from the cavity. The fluid may be removed through the use of osmotic or electro-osmotic cells, or through a one-way valve.

Document US 2006/0235358 A1 discloses a method and apparatuses to increase tissue oxygenation through a patient's damaged tissue to heal wounds. The method teaches to create an enclosed environment, immediately outside a patient's damaged tissue, in which the oxygen concentration is reduced to prompt an outward supply of oxygen flows from blood capillaries to the damaged tissue and eventually out of the skin. The apparatus taught in this document use two different ways to reduce the oxygen: oxygen scavengers and burning off the oxygen.

SUMMARY

In view of the foregoing, the invention provides a controlled pressure device according to claim 1 and a non-therapeutic method of treating a tissue site according to claim 14.

A controlled pressure device includes a reactor housing element and a reactor. The reactor housing element is configured to at least partially define an at least substantially air-tight enclosed volume around a tissue site when fixed in space in relation to the tissue site. The reactor is positioned in the enclosed volume and is configured to react with a selected gas found in air. The reactor consumes the selected gas within the enclosed volume.

Another example of a controlled pressure device can include a reactor housing element and a reactor. The reactor housing element is configured to at least partially define an at least substantially air-tight enclosed volume around a tissue site when fixed in space in relation to the tissue site, and the enclosed volume has a determined volume (DV). The reactor is positioned in the enclosed volume and is configured to react with a selected gas found in air. The reactor consumes the selected gas within the enclosed volume. The reactor is configured having a predetermined scavenging capacity (SC) for the selected gas. The controlled pressure device is configured to have a maximum leakage rate (LR) when affixed to a subject's skin for air entering the enclosed volume. The controlled pressure device is also configured to a minimum wear time (MWT) and is configured according to the following relationship: SC > DV*(% of selected gas in air) + LR*(% of selected gas in air)*MWT.

In view of the foregoing, method of treating a tissue site includes removing a release layer from a reactor housing element. The method further includes placing the reactor housing element, a reactor and a liquid impermeable - air permeable membrane over the tissue site. The method also includes affixing the reactor housing element with respect to skin around the tissue site to define an at least substantially air-tight enclosed volume around the tissue site.

BRIEF DESCRIPTION OF THE DRAWINGS

FIG. 1 is a schematic cross-sectional view of a controlled pressure device.

FIG. 2 is a schematic exploded perspective view of a controlled pressure device.

FIGS. 3 and 4 are schematic depictions of alternative reactor embodiments that can be used with the controlled pressure device shown in FIG. 1.

FIGS. 5-7 are schematic depictions of powered components that can be used with the controlled pressure device shown in FIG. 1.

FIGS. 8 and 9 are flow diagrams depicting a method of treating a tissue site.

DETAILED DESCRIPTION

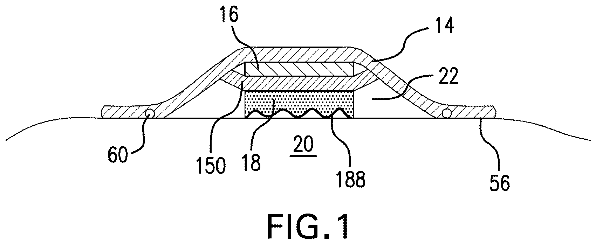

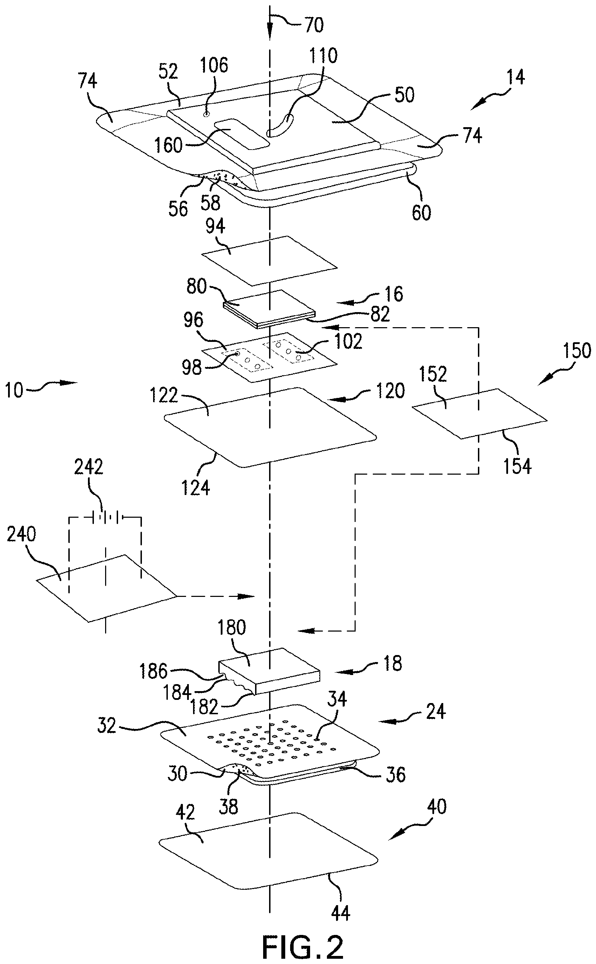

FIG. 1 depicts a controlled pressure device 10 including a reactor housing element 14 and a reactor 16. The controlled pressure device 10 can be used in conjunction with a cosmetic liquid or cream for non-therapeutic tissue treatment. For example, the controlled pressure device 10 can include or be used in conjunction with a pad, which can be wetted or impregnated with a cosmetic liquid or cream, and will be hereinafter referred to as a cosmetic pad 18. The controlled pressure device 10 can be positioned at a tissue site 20 to enhance non-therapeutic tissue treatment including, but not limited to, reduction of skin wrinkles. The reactor housing element 14 is configured to at least partially define an at least substantially air-tight enclosed volume 22 around the tissue site 20 when affixed in space in relation to the tissue site 20. FIG. 1 depicts the reactor housing element 14 in contact with the tissue (e.g., skin) around the tissue site 20, however, the reactor housing element 14 can be fixed in space in relation to the tissue site 20 without being in direct contact with the tissue around the tissue site 20. For example, an intermediate membrane or layer, such as a skin contacting element 24 (FIG. 2), could be interposed between the reactor housing element 14 and the tissue (e.g., skin) around the tissue site 20, although the reactor housing element 14 is fixed in space in relation to the tissue site 20. The reactor 16 positioned in the enclosed volume 22 reacts with a selected gas found in air and consumes the selected gas within the enclosed volume 22. The cosmetic liquid or cream is also located in the enclosed volume 22.

With reference to FIG. 2, the controlled pressure device 10 can includes the skin contacting element 24. The skin contacting element 24 includes a skin contacting side 30 that is adherable to a subject's skin and an interface side 32, which is opposite to the skin contacting side. In the illustrated embodiment, the skin contacting element 24 is shown as a separate element from the reactor housing element 14. In an alternative arrangement, the skin contacting element 24 can be provided as part of the reactor housing element 14, e.g., the skin contacting element 24 can be integrally formed with the reactor housing element 14 or connected with the reactor housing element 14 at the manufacturing facility. The skin contacting element 24 can be made from a thin sheet-like membrane, for example using a roll-to-roll process. In the illustrated embodiment, the skin contacting element 24 also includes openings 34 extending through the skin contacting element 24 from the skin contacting side 30 to the interface side 32. The openings 34 can be located over the tissue site 20. In conjunction with a skin contacting gasket 36, the skin contacting element 24 is configured to provide a seal around the tissue site 20 to allow for topical negative pressure to be applied at the tissue site 20. If desired, the skin contacting element 24 can include an opening that is larger than each of the openings 34 depicted in FIG. 2. In this example, the opening 34 can be dimensioned to fit around the tissue site 20. The opening 34 in this example can be similarly shaped to the cosmetic pad 18 so that the skin contacting element 24 and the skin contacting gasket 36 provide a seal around the tissue site 20 (FIG. 1) and the cosmetic pad 18. If desired, the skin contacting element 24 need not include the opening(s) 34, and in this example, the cosmetic pad 18 can be positioned beneath the skin contacting side 30 so as to come in contact with the subject's skin at the tissue site 20.

Both the skin contacting side 30 and the interface side 32 are generally flat or planar. In the illustrated embodiment, the interface side 32 is the upper side of the skin contacting element 24 and the skin contacting side 30 is the lower side of the skin contacting element. Adhesive 38 (depicted schematically in FIG. 2) can provided on the skin contacting side 30 to allow the skin contacting side 30 to adhere to the subject's skin around the tissue site 20.

The skin contacting gasket 36 can be provided on the skin contacting side 30 of the skin contacting element 24 or simply be positioned between the skin contacting side 30 and the subject's skin. The skin contacting gasket 36 can be made from a hydrogel to further promote sealing around the tissue site 20 to promote the topical negative pressure at the tissue site 20.

A release layer 40 can be provided with the controlled pressure device 10 to protect the skin contacting side 30 of the skin contacting element 24. The release layer 40 can be similar to known release layers and include an upper side 42 that is releasable from the skin contacting side 30 of the skin contacting element 24. The release layer 40 also includes a lower surface 44 opposite the upper side 42. As mentioned above, the reactor 16 reacts with a selected gas found in air and consumes the selected gas within the enclosed volume 22. In some embodiments, the release layer 40 can provide an air-tight barrier such that air is precluded from access to the reactor 16 until after the release layer 40 is removed from the skin contacting element 24. In embodiments where the skin contacting element 24 may not be provided, the release layer 40 can cooperate with the reactor housing element 14 in a similar manner, and the release layer 40 can provide an air-tight barrier such that air is precluded from access to the reactor 16 until after the release layer 40 is removed from the reactor housing element 14.

With reference back to FIG. 1, the reactor housing element 14 at least partially defines the enclosed volume 22 around the tissue site 20 when fixed in space in relation to the tissue site 20. With reference to the embodiment depicted in FIG. 2, the reactor housing element 14 cooperates with the skin contacting element 24 to at least partially define the enclosed volume 22 (see FIG. 1) around the tissue site 20, for example, when the skin contacting side 30 of the skin contacting element 24 is in contact with and adhered to the subject's skin and the reactor housing element 14 is affixed to the skin contacting element 24. The reactor housing element 14 can be formed with a hood 50 and a lower peripheral section 52 that at least partially surrounds the hood 50. In the illustrated embodiment, the lower peripheral section 52 entirely surrounds the hood 50, which is raised in relation to the lower peripheral section 52 so as to define the enclosed volume 22 around the tissue site 20. The reactor housing element 14 can also be formed without the hood 50, i.e., the reactor housing element 14 could initially be planar. Similar to the skin contacting element 24, the reactor housing element 14 can be made from a thin sheet-like membrane, for example using a roll-to-roll process. The reactor housing element 14 can be made from a flexible material that is similar to or the same flexibility as the skin contacting element 24, which allows the controlled pressure device 10 to conform to the skin around the tissue site 20. When the reactor housing element 14 is affixed to the skin contacting element 24, the reactor 16 is located between the reactor housing element 14 and the cosmetic pad 18 and the skin contacting element 24. Since the reactor housing element 14 is made from a flexible material in this example, the section of the reactor housing element 14 that is not in contact with the skin contacting element 24 is raised or offset from the tissue site 20 so as to form the hood 50.

The reactor housing element 14 can be made from a material that is air impermeable so that air is precluded or greatly inhibited from entering into the enclosed volume 22 as the reactor 16 consumes a selected gas within the enclosed volume thus reducing gas pressure within the enclosed volume. The reactor housing element 14 could also be made from materials that are impervious to particular gasses (e.g., oxygen) and pervious to other gases (e.g., nitrogen). The reactor housing element 14 can be formed of a material that is at least partially gas permeable for selected gasses (e.g., nitrogen gas permeable) to exhaust the selected gas from the tissue site 20 to atmosphere. The reactor housing element 14 includes a lower surface 56. An adhesive 58 (depicted schematically in FIG. 2) can be applied to the lower surface 56. With reference to FIG. 1, the lower surface 56 contacts the skin around the tissue site 20 and the adhesive 58 adheres the reactor housing element 14 to the skin around the tissue site 20. A reactor housing element gasket 60, which can be a hydrogel gasket similar to the skin contacting gasket 36 described above, can be provided between the lower surface 56 of the reactor housing element 14 and the skin to further seal between the reactor housing element 14 and the skin to preclude air migration between the interface between the lower surface 56 the skin.

With reference to FIG. 2, the reactor housing element 14 can affix to the interface side 32 of the skin contacting element 24 providing a substantially air-tight seal between the reactor housing element 14 and the skin contacting element 24. The adhesive 58 applied to the lower surface 56 can affix the reactor housing element 14 to the skin contacting element 24. The reactor housing element gasket 60 can be provided between the lower surface 56 of the reactor housing element 14 and the interface side 32 of the skin contacting element 24 to further seal between the reactor housing element 14 and the skin contacting element 24 to preclude air migration between the interface between the lower surface 56 of the reactor housing element 14 and the interface side 32 of the skin contacting element 24.

The reactor 16 is positioned in the enclosed volume 22 and is configured to react with one of more selected gases (e.g., nitrogen, oxygen, carbon dioxide) found in air to consume the selected gas within the enclosed volume 22, which can reduce gas pressure within the enclosed volume 22. The reduction in gas pressure within the enclosed volume 22 can result in a partial vacuum being formed in the enclosed volume 22, which can result in a downward force (in the direction of arrow 70) being applied to the hood 50 of the reactor housing element 14. The reactor housing element 14 can be configured such that the downward force (in the direction of arrow 70) on the hood 50 (or the top of the reactor housing element 14) results in an outward force (normal to the direction of arrow 70) on the lower peripheral section 52. Since the reactor housing element 14 is affixed to the skin contacting element 24, which is adhered to the subject's skin, or directly to the skin (see FIG. 1) the outward force in the direction normal to arrow 70 can result in small strains and stresses being applied to the patient's skin around the tissue site 20. These strains and stresses being applied to the patient's skin can be beneficial for the introduction of the cosmetic liquid or cream impregnated within the cosmetic pad 18 as well as providing a massaging effect of the skin. Where the reactor housing element 14 is made from a relatively more rigid material with respect to the skin contacting element 24, for example, flexible regions 74 can be provided in the lower peripheral section 52 to facilitate outward movement of the reactor housing element 14 with respect to the tissue site 20 to allow for the small strains and stresses to be applied to the subject's skin at the tissue site 20.

The reactor 16 is positioned in the enclosed volume 22 and is configured to react with the selected gas (e.g., nitrogen, oxygen, carbon dioxide) found in air. As the reactor 16 consumes the selected gas within the enclosed volume 22, the gas pressure within the enclosed volume 22 is reduced. For example, where the reactor 16 consumes oxygen, there can be an approximate 20% reduction from atmospheric pressure in the enclosed volume 22. An example of a reactor 16 that can be used in the controlled pressure device is described in US 2014/0109890 A1. US 2014/0109890 A1 describes an oxygen based heater; however, the oxygen based heater described in US 2014/0109890 A1 can be used as the reactor 16 to consume oxygen within the enclosed volume 22 thus producing a partial vacuum within the enclosed volume 22. In this example, the reactor 16 includes a reducing agent, a binding agent on a reactor substrate 80 and an electrolyte solution, which can be provided in an electrolyte impregnated pad 82. The reducing agent on the reactor substrate 80 can be zinc, aluminum, or iron, for example.

As mentioned above, the release layer 40 can operate as an air-tight barrier such that the selected gas (e.g., nitrogen, oxygen, carbon dioxide) is precluded from access to the reactor 16 until after the air-tight barrier, which in this instance is the release layer 40, is removed from the controlled pressure device 10. Alternatively, the controlled pressure device 10 can include a package, which is shown in FIG. 2 as including an upper layer 94 and a lower layer 96. The upper layer 94 affixes to the lower layer 96 enclosing the reactor substrate 80 in between the upper layer 94 and the lower layer 96 so as to provide an air-tight seal so that the selected gas is precluded from access to the reactor 16. In this example including the package, the upper layer 94 or the lower layer 96 can operate as an air tight barrier in that removal of the upper layer 94 from the lower layer 96, or vice versa, allows the selected gas access to the reactor 16 so that the selected gas can be consumed by the reactor 16. Alternatively, at least one of the layers (the lower layer 96 in the illustrated embodiment) can include openings 98 and a seal layer 102 can be affixed in an air-tight manner to the lower layer 96 covering the openings 98. Removal of the seal layer 102 from the lower layer 96 exposes the reactor 16 to the selected gas, which allows the reactor to consume the selected gas within the enclosed volume 22. The surface area of the openings 98 can be appropriately sized to control the flow of the selected gas through the openings to sustain the chemical reaction of the reactor 16 to the desirable time limit for maintaining topical negative pressure on the tissue site 20 for the desired duration. Moreover, multiple seal layers 102 can be provided so that removal of one or a selected few of the seal layers (while other seal layers are still affixed to the lower layer 96) can also be provided to limit the flow of the selected gas toward the reactor 16. Additionally, to further control the pressure within the enclosed volume, the controlled pressure device 10 can include a pressure relief valve 106 on the reactor housing element 14. The pressure relief valve 106 can allow for selected communication between the enclosed volume 22 and ambient. The pressure relief valve 106 can be operated when a predetermined pressure differential exists between the enclosed volume 22 and ambient. Moreover, the pressure relief valve 106 can be configured to open when the temperature within the enclosed volume exceeds a desired predetermined temperature.

To also further control the pressure within the enclosed volume 22, the controlled pressure device 10 can be configured to allow an operator to change the size of the enclosed volume 22 while the reactor 16 consumes the selected gas in the enclosed volume, thus varying the gas pressure within the enclosed volume 22. For example, a tab 110 can be affixed to the reactor housing element 14, and the operator can pull the tab 110 to move the reactor housing element away from the tissue site 20. In FIG. 2, the tab 110 is affixed to the hood 50. When the enclosed volume 22 is under negative pressure, the hood 50 will tend to travel toward the tissue site 20. By pulling on the tab 110, the hood 50 can be moved away from the tissue site 20 and air can enter the enclosed volume 22, for example by leaking past the gasket 36 or a small pre-configured leakage path can be provided. Air entering the enclosed volume 22 can increase the pressure within the enclosed volume 22 until the selected gas (e.g., oxygen) is consumed by the reactor 16. Such a configuration can allow for a cycling of pressure in the enclosed volume 22.

The controlled pressure device 10 can also be packaged such that the reactor housing element 14 and the reactor 16 are packaged separately from the skin contacting element 24, for example. In such an embodiment, a reactor housing element release layer 120 can be provided. The reactor housing element 14 release layer 120 can be similar to the release layer 40 having an upper side 122 that is releasable from the lower surface 56 of the reactor housing element 14. The reactor housing element release layer 120 further includes a lower surface 124 that is opposite to the upper side 122. The reactor housing element release layer 120 can also be affixed to the lower layer 96, so that removal of the reactor housing element release layer 120 from the reactor housing element 14 results in removal of the lower layer 96 from the upper layer 94, thus exposing the reactor 16 to air. Alternatively, the reactor housing element release layer 120 can be affixed to the seal layer 102 such that removal of the reactor housing element release layer 120 from the reactor housing element 14 results in removal of the seal layer 102 from the lower layer 96, thus exposing the reactor 16 to air via the openings 98 provided in the lower layer 96.

Where the controlled pressure device 10 is packaged where the reactor housing element 14 and the reactor 16 are separate from the skin contacting element 24, an upper skin contacting element release layer (not shown) can be provided. The upper skin contacting element release layer can be similar to the release layer 40. Alternatively, no upper skin contacting element release layer need be provided.

The controlled pressure device 10 further includes a liquid impermeable-air permeable membrane 150 interposed between the reactor 16 and the cosmetic liquid or cream, which is impregnated in the cosmetic pad 18 in the illustrated embodiment. The size (area) of the liquid impermeable-air permeable membrane 150 can depend on the location of the liquid impermeable-air permeable membrane 150 within the controlled pressure device 10. If the cosmetic liquid or cream were to come in contact with the reactor 16, the chemical reaction of the reactor 16 when coming in contact with the selected gas may be detrimentally impacted. As such, the liquid impermeable-air permeable membrane 150 allows air flow within the enclosed volume 22 while precluding the cosmetic liquid or cream from coming into contact with the reactor 16.

The liquid impermeable-air permeable membrane 150 can be located in different locations on the controlled pressure device 10. In one example depicted in FIG. 2, the liquid impermeable-air permeable membrane 150 can be located between the reactor substrate 80 and the lower layer 96 making up the air-tight package for the reactor 16. So, once the lower layer 96 (or the seal layer 102) is removed, air can gain access to the reactor 16, but the cosmetic liquid or cream would be precluded from traveling through the liquid impermeable-air permeable membrane 150. In another example, the liquid impermeable-air permeable membrane 150 can be located between the lower layer 96 (or the seal layer 102) making up the air-tight package for the reactor 16 and the cosmetic liquid or cream, which can be impregnated in the cosmetic pad 18. More generally, and as depicted in FIG. 1, the liquid impermeable-air permeable membrane 150 can be located between the reactor 16 and the cosmetic liquid or cream, which would be impregnated in the cosmetic pad 18. As depicted in FIG. 1, the liquid impermeable-air permeable membrane 150 can be connected with the reactor housing element 14 and preclude the cosmetic liquid or cream from passing through the liquid impermeable-air permeable membrane 150 while allowing air to travel through the liquid impermeable-air permeable membrane 150 so that the selected gas can be removed from the enclosed volume 22. If desired, the liquid impermeable-air permeable membrane 150 could surround the reactor 16 and preclude the cosmetic liquid or cream from passing through the liquid impermeable-air permeable membrane 150 while allowing air to travel through the liquid impermeable-air permeable membrane 150.

In an embodiment where the reactor housing element 14 and the reactor 16 are packaged separately from the skin contacting element 24, the release layer 40 can be removed from the skin contacting element 24. The skin contacting element 24 can be pressed against a subject's skin around the tissue site 20 and adhesive 38 on the skin contacting side of the skin contacting element 24 can adhere the skin contacting element 24 to the subject's skin. The skin contacting gasket 36 can surround the tissue site 20 to promote an air-tight seal between the skin contacting element 24 and the subject's skin around the tissue site 20. The cosmetic liquid or cream, which is located within the cosmetic pad 18 in the illustrated embodiment, is then brought into contact with the tissue site 20 by traveling through the openings 34.

The reactor housing element release layer 120 can be removed from the reactor housing element 14. Removal of the reactor housing element release layer 120 from the reactor housing element 14 can expose the reactor 16 to air by removal of the lower layer 96, the seal layer 102, or the reactor housing element release layer 120 may be affixed in an air-tight manner to the reactor housing element 14 so that air is precluded from access to the reactor 16 until after the reactor housing element release layer 120 is removed from the reactor housing element 14. After removal of the reactor housing element release layer 120 from the reactor housing element 14, the lower surface 56 of the reactor housing element 14 can be brought in contact with an upper surface 152 of the liquid impermeable-air permeable membrane 150, which can have its lower surface 154 affixed to the interface side 32 of the skin contacting element 24. Alternatively, where the liquid impermeable-air permeable membrane 150 is positioned between the reactor substrate 80 and the lower layer 96, the lower surface 56 of the reactor housing element 14 can be brought in contact with the interface side 32 of the skin contacting element 24. When the reactor housing element 14 is brought into contact with the liquid impermeable-air permeable membrane 150 and/or the skin contacting element 24, the reactor 16 consumes the selected gas found within the enclosed volume 22. A chemical reaction occurs where heat can be generated, and a pressure reduction with respect to atmospheric pressure occurs by consumption of the selected gas.

In a non-claimed embodiment, the reactor 16 can heat the enclosed volume 22 above ambient, which can provide a therapeutic effect for use with some cosmetic liquids or creams. In addition, but not part of the claimed subject-matter, suction can be applied to the tissue site thus stretching the subject's skin at the tissue site, which can also have a therapeutic effect with certain cosmetic liquids or creams. Moreover, the non-claimed application of topical negative pressure at a particular tissue site can also have a therapeutic effect.

The controlled pressure device 10 can be designed with certain parameters. As an example, assuming the tissue site 20 of about 10cm x 20cm and an offset of the hood 50 (or the top of the reactor housing element 14) of about 2.5cm from the tissue site 20 results in the enclosed volume 22 of 500mL. Assuming that the cosmetic liquid or cream and the solid components of the cosmetic pad 18 in addition to the reactor 16 and any other solid components (e.g., the liquid impermeable-air permeable membrane 150) within the enclosed volume 22 account for 100mL within the enclosed volume 22, this leaves 400mL air in the enclosed volume. 400mL of air results in about 320mL of nitrogen and 80mL of oxygen within the enclosed volume 22 prior to the application of a partial vacuum resulting from the reactor 16 consuming a selected gas in the air within the enclosed volume 22. One gram (1g) of zinc (Zn) will consume about 170mL at standard temperature and pressure (STP) of oxygen ($O_2$), which is the amount of oxygen in about 850mL (STP) of normal dry air. Although the skin contacting gasket 36 and the reactor housing element gasket 60 can be provided, there will likely be leakage of ambient air into the enclosed volume 22 past the gaskets 36, 60 and possibly diffusion through the reactor housing element 14 and the skin contacting element 24. For the purposes of this disclosure, both leakage past interfaces (e.g., leakage around the skin contacting gasket 36) and diffusion (e.g., diffusion through the reactor housing element 14) will be referred to as leakage. The gaskets 36, 60, the skin contacting element 24 and reactor housing element 14 are configured to have a maximum leakage rate of air into the enclosed volume 22 from ambient. For example, a maximum leakage rate of 1mL(STP)/hour of air into the enclosed volume from ambient results in 0.2mL(STP) of oxygen/hour. Since 1g of zinc consumes 170mL of oxygen (STP), 1g of zinc provides an adequate amount of a reducing agent to result in a 20% reduction from normal atmospheric pressure within the enclosed volume 22 for an extended period of time, e.g., well over 72 hours. The controlled pressure device 10 will likely be worn for a much shorter period of time and the tissue site 20 being treated may be much smaller than 10cm x 20cm. It can be seen that a very small reactor 16, e.g., one able to accommodate less than 1g of zinc, can be used in the controlled pressure device 10.

In view of the foregoing, the controlled pressure device 10 can be configured as follows. The reactor 16 can be configured to have a predetermined scavenging capacity ("SC"), which relates to the volume of the selected gas that the reactor 16 is configured to consume. For example, as mentioned above 1g of zinc will consume about 170mL of oxygen (STP), so the scavenging capacity would be 170mL. The enclosed volume 22 can have a determined volume ("DV") based on the area of the tissue site 20, the size of the reactor housing element 14, the offset of the hood 50 (or top of the reactor housing element 14) from the tissue site 20, taking into account the cosmetic liquid or cream and the solid components of the cosmetic pad 18 in addition to the reactor 16 and any other solid components within the enclosed volume 22. For example, the determined volume of 400mL was discussed above. Also, the controlled pressure device 10 can be configured to have a maximum leakage rate (LR) for air entering the enclosed volume 22. In addition, the controlled pressure device 10 can be configured to have a minimum wear time ("MWT"), which relates to the minimum amount of time that the controlled pressure device 10 is configured to be worn. Assuming that it is desirable to have the reactor 16 consume, or scavenge, the selected gas for the entire minimum wear time, the controlled pressure device can be configured in view of the following relationship:

$$SC > DV*(\% \text{ of selected gas in air}) + LR*(\% \text{ of selected gas in air})*MWT.$$

The scavenging capacity can be determined to provide a relatively small reactor 16 in relation to the reactor housing element 14 and the tissue site 20 to be treated. The determined volume can be determined to provide a relatively small reactor 16 and reactor housing element 14 in view the tissue site 20 to be treated. The maximum leakage rate for air entering the enclosed volume 22 should be reduced as much as is practical; however, there may be some circumstances in which a predetermined amount of leakage is desirable, for example where cycling of pressure within the enclosed volume 22 is desired. For example, the maximum leakage rate for air entering the enclosed volume 22 can be less than 10mL/hour, and preferably less than 1 or 2mL/hr. The minimum wear time can be determined based on the desired amount of time the topical negative pressure is to be applied to the tissue site 20. It may be desirable to include a safety factor (e.g., a multiplier on the right side of the relationship above) to accommodate for manufacturing tolerances, differences among tissues sites and subjects placing the controlled pressure device 10 on the tissue site 20.

As the reactor 16 consumes the selected gas found in air within the enclosed volume 22, an exothermic reaction occurs such that there is an increase in temperature of the reactor 16. As such, the reactor 16 can operate as a heater to heat the cosmetic liquid or cream impregnated in the cosmetic pad 18. This can change the viscosity of the cosmetic liquid or cream, which can facilitate entry of the cosmetic liquid or cream into skin pores.

The reactor housing element 14 could also be provided with a removable section 160 that when removed could provide ambient air access to the reactor 16 or an additional reactor located beneath the reactor housing element 14. For example, the removable section 160 can be affixed to the upper layer 94 of the package in which the reactor substrate 80 is located. Removal of the removable section 160 can result in removal of at least a portion of the upper layer 94 thus exposing the reactor substrate 80 to ambient air, which would result in an exothermic reaction. Alternatively, an additional reactor could be located beneath the reactor housing element 14 and removal of the removable section 160 can result in removal of at least a portion of the package (similar to the package made up of the upper layer 94 and the lower layer 96) thus exposing the additional reactor to ambient air. The additional reactor can also heat the gas in the enclosed volume 22, which can heat the tissue site 20.

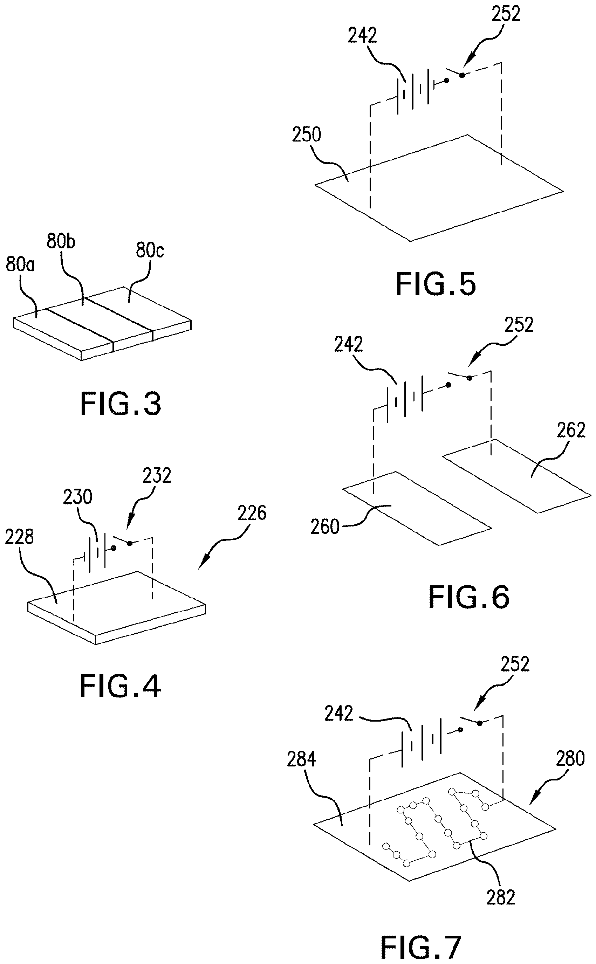

Different types of reactors could be used to provide topical negative pressure inside the enclosed volume 22 of the controlled pressure device 10. FIG. 3 depicts multiple reactor substrates or multiple regions on the same reactor substrate, which are depicted as reactor elements 80a, 80b and 80c each having different chemical properties and/or characteristics. For example, the first reactor element 80a can be configured to begin consuming oxygen after being exposed to oxygen for a very short (first) period of time t1, e.g., nearly instantaneously. The second reactor element 80b can be configured to begin consuming oxygen after being exposed to oxygen for a longer (second) period of time t2, and the third reactor element 80c can be configured to begin consuming oxygen after being exposed to oxygen for an even longer (third) period of time. Alternatively, the second reactor element 80b can be configured with a delayed reaction time to begin consuming oxygen after the first reactor element 80a has been exhausted and no longer consumes oxygen. Similarly, the third reactor element 80c can be configured with a delayed reaction time to begin consuming oxygen after the first reactor element 80a and the second reactor element 80b have both been exhausted and no longer consume oxygen. Such a configuration can allow for a cycling of pressure in the enclosed volume 22.

In lieu of the reactor 16 made up of the reactor substrate 80, the reactor 16 may be one or any combination of a zinc-based chemical pump, electro-chemical pumps, vacuum-on-demand devices (referred to herein as VOD), electrolyzers, pressure-reducing solid state devices, oxygen absorbing iron packets, or getters of zirconium titanium, vanadium iron, lithium, lithium metal, magnesium, calcium, lithium barium combinations, zinc-air battery, zinc-air battery components or other materials highly reactive with the selected gases, for example, nitrogen, carbon dioxide and oxygen gases found in skin tissue environments.

FIG. 4 depicts another reactor 226 that can be used in place of the reactor 16 shown in FIG. 1. WO 2015/054040 A1 describes an electrochemical cell that is adapted to consume gases, i.e., air or its gaseous non-noble constituents, within an enclosure via an electrochemical reaction. This consumption of gas within a sealed enclosure forms a partial vacuum. The controlled pressure device 10 can include such a reactor 226 having an electrochemical cell 228 that lowers the pressure within the enclosed volume 22 through an electrochemical reaction that takes place when a voltage is applied to the electrochemical cell 228 by a power source 230 (depicted schematically in FIG. 4). Operation of electrochemical cell 228 in this example can also be achieved by controlling the current supplied to the electrochemical cell 228 by the power source 230, for example by providing a switch 232 that can be operated by the user. The power source 230 can be located in the enclosed volume 22 or be provided outside the controlled pressure device 10 (e.g. on the reactor housing element 14). The switch 232 can be provided outside the controlled pressure device 10 (e.g. on the reactor housing element 14).

In an example where the reactor is a VOD device, a VOD is a solid state electrochemical cell, which when charged with a low voltage, produces a highly reactive material that captures gases present in the atmosphere and when sealed in an air tight system can form a partial vacuum. In a VOD device, metal is deposited to grow dendrites as a voltage is applied across electrodes of the VOD device and lithium salt electrolyte, charging the VOD device. Similar to charging a battery, electrons are moved from layer to layer to form metallic lithium.

In an example where the reactor is a getter, a getter, as known in the art, is a deposit of reactor material that is used for initiating and maintaining a partial vacuum. When gas molecules strike the getter material, particular gas molecules (i.e., those of the selected gas) combine with the getter chemically or by adsorption. Thus the getter removes the selected gas from the evacuated space until the active material is exhausted.

A reactor having a self-regulating oxygen getter powered by zinc-air battery technology may be used in lieu of the above-described reactor 16 made up of the reactor substrate 80. Zinc-air batteries can react to control or reduce the oxygen levels in sealed site and thus self-regulate a reduced pressure of approximately 0.8 bars. If the zinc-air battery components are configured as a working zinc-air battery, the battery voltage will drop when the oxygen has been depleted and the desired partial vacuum pressure will have been achieved. This drop in voltage may be used to indicate that the desired partial vacuum has been achieved. For example, a 675 size hearing aid zinc-air battery is rated at 620 mAh, occupies 0.5 mL volume, and weighs 1.9g. A 675 zinc-air battery can remove more than 150 times its volume of oxygen.

The cosmetic pad 18 can be made from a blend of polyester and cellulose fibers, polypropylene fibers, or other suitable non-woven polymeric material. The cosmetic liquid or cream can include at least one of a moisturizer, dimethylaminoethanol (DMAE), Acetyl hexapeptide-8, Acetyl hexapeptide-3, retinol, ubiquinone, dithiolane-3-pentanic acid, alpha-hydroxy acid, alpha lipoic acid, salicylic acid, hydrocortisone, topical botulinum cream, and hyaluronic acid. The cosmetic pad 18 can include top surface 180 and a bottom surface 182, which is opposite the top surface 180. The bottom surface 182 can be undulated having hills 184 and valleys 186, which can allow the cosmetic pad 18 to act as a skin massaging element. An additional membrane 188 could be provided and located beneath the cosmetic pad 18 to act as the skin massaging element. This additional membrane 188 would also be positioned adjacent to the tissue site 20. This additional membrane 188 could also include an undulated bottom surface, similar to the bottom surface 182 having hills 184 and valleys 186. This additional membrane 188 would also include openings to allow the cosmetic liquid or cream to pass through the openings to the tissue site 20. When a partial vacuum is provided in the enclosed volume 22, the skin can be drawn towards the undulated surface and the skin can conform to the hills 184 and valleys 186. Accordingly, small strains and stresses can applied to the patient's skin at the tissue site 20 providing a massaging effect.

The controlled pressure device 10 can also include additional powered components, which is shown as a powered component 240 that is schematically depicted in FIG. 2. Each powered component 240, which are described later with reference to FIGS. 5 - 7, can be electrically connected with a power source 242 (depicted schematically in FIG. 2) which, for example, can be a zinc-air battery exposed to ambient or a zinc MnO2 battery, electrically connected with the powered component 240. When using a zinc-air battery as the power source 242, the power source 242 would be located outside of the enclosed volume 22 or a section of the reactor housing element 14 or the skin contacting element 24 could be removable to allow ambient air to contact the zinc-air battery.

FIG. 5 depicts a heater 250, which is one example of a powered component 240 that can be used in the controlled pressure device 10, electrically connected with the power source 242. The heater 250 can be a thin film heater or thin film nano wire heater. The heater 250 can heat the cosmetic liquid or cream impregnated in the cosmetic pad 18. The heater 250 can be positioned adjacent to the cosmetic pad 18. A switch 252 can be provided to control power to the heater 250. The heater 250 can also heat the gas in the enclosed volume 22, which can heat the tissue site 20.

FIG. 6 depicts a first electrode 260 and a second electrode 262 electrically connected with the power source 242. The electrodes are another example of a powered component 240 that can be used in the controlled pressure device 10. The switch 252 can be provided to control power to the electrodes 260, 262. The first electrode 260 can be positioned on a first side of the tissue site 20 and in contact with the skin, and the second electrode 262 can be positioned on a second, opposite, side of the tissue site 20 and in contact with the skin. The electrodes 260, 262 can be used to provide electrical stimulation to the tissue site 20.

FIG. 7 depicts a light source 280, which is another example of a powered component 240 that can be used in the controlled pressure device 10. The light source 280 can include a plurality of LEDs 282 mounted on a flexible substrate 284. The LEDs 282 are electrically connected with the power source 242. The flexible substrate 284 can be placed adjacent to the skin contacting element 24, which can include larger openings 34, which can allow light to pass for phototherapy.

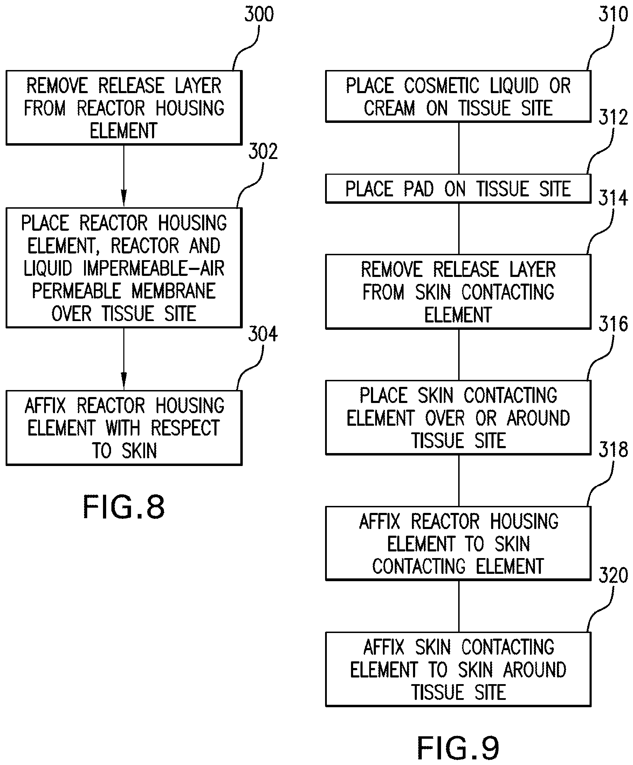

FIG. 8 depicts a method of treating tissue using negative pressure. Although the FIG. 8 shows a specific order of method steps, the order of the steps may differ from what is depicted. Also two or more steps may be performed concurrently or with partial concurrence. The method will be described with reference to the controlled pressure device 10 depicted in FIGS. 1 and 2, however, the method can be practiced with other devices.

At 300, the reactor housing element release layer 120 is removed from the reactor housing element 14. Removal of the reactor housing element release layer 120 from the reactor housing element 14 can allow air to contact the reactor 16, which can begin the reaction in which oxygen (or another selected gas in air) is being consumed by the reactor 16. At 302, the reactor housing element 14, the reactor 16 and the liquid impermeable - air permeable membrane 150 are placed over the tissue site 20. At 304, the reactor housing element 14 is affixed with respect to the skin around the tissue site 20 to define an at least substantially air-tight enclosed volume 22 around the tissue site. The reactor 16 can consume the selected gas (e.g., oxygen) from the enclosed volume 22 thus reducing the gas pressure in the enclosed volume 22.

FIG. 9 depicts other steps in a method of treating tissue using negative pressure. For example, the method may include, at 310, placing a cosmetic liquid or cream on the tissue site 20. The cosmetic liquid or cream may be placed on the tissue site 20 prior to placing the reactor housing element 14, the reactor 16 and the liquid impermeable - air permeable membrane 150 over the tissue site 20, which occurs at step 302.

The method of treating tissue using negative pressure may also include, at 312, placing a pad 18 impregnated or wetted with a cosmetic liquid or cream over the tissue site 20. The pad 18 can be connected with the reactor housing element 14 in such a manner that the pad 18 is placed on the tissue site 20 while placing the reactor housing element 14, the reactor 16 and the liquid impermeable - air permeable membrane 150 over the tissue site 20.

The method of treating tissue using negative pressure may also include, at 314, removing the release layer 40 from the skin contacting element 24, and, at 316, placing the skin contacting element 24 over or around the tissue site 20. The skin contacting element 24 can be placed over or around the tissue site 20 prior to placing the reactor housing element 14, the reactor 16 and the liquid impermeable - air permeable membrane 150 over the tissue site 20, which occurs at step 302. As such, affixing the reactor housing element 14 with respect to skin around the tissue site, which occurs at step 304, can further include affixing the reactor housing element 14 to the skin contacting element 24, at 318. The method may also include affixing the skin contacting element 24 to the skin around the tissue site 20, at 320.

**Claims**

1. A controlled pressure device (10) for tissue treatment, the device (10) comprising:

   a reactor housing element (14) configured to at least partially define an at least substantially air-tight enclosed volume (22) around a tissue site (20) when fixed in space in relation to the tissue site (20); and
   a reactor (16) positioned in the enclosed volume (22) and configured to react with a selected gas found in air, wherein the reactor (16) consumes the selected gas within the enclosed volume (22); **characterised by**
   a cosmetic liquid or cream located in the enclosed volume (22).

2. The controlled pressure device (10) of claim 1, wherein the cosmetic liquid or cream includes at least one of a moisturizer, dimethylaminoethanol (DMAE), Acetyl hexapeptide-8, Acetyl hexapeptide-3, retinol, ubiquinone, dithiolane-3-pentanic acid, alpha-hydroxy acid, alpha lipoic acid, salicylic acid, hydrocortisone, topical botulinum cream, and hyaluronic acid.

3. The controlled pressure device (10) of claim 1, further comprising a pad (18) impregnated with the cosmetic liquid or cream.

4. The controlled pressure device (10) of claim 1, wherein the reactor (16) includes a reactor substrate (80), a reducing agent, a binder, and an electrolyte solution.

5. The controlled pressure device (10) of claim 1, wherein the reactor (16) is configured to cycle gas pressure in the enclosed volume (22),

   wherein the reactor (16) includes a plurality of reactor elements (80a, 80b, 80c), or
   the reactor (16) includes a first reactor element (80a) configured to begin consuming oxygen after being exposed to oxygen for a first period of time and a second reactor element (80b) configured to begin consuming oxygen after being exposed to oxygen for a second period of time, which is greater than the first period of time, or
   the reactor (16) includes a first reactor element (80a) and a second reactor element (80b), wherein the second reactor (80b) element is configured with a delayed reaction time to begin consuming oxygen after the first reactor element (80a)has been exhausted and no longer consumes oxygen.

6. The controlled pressure device (10) of claim 1, wherein the reactor housing element (14) includes a hood (50) and a lower peripheral section (52) at least partially surrounding the hood (50), the reactor housing element (14) being configured such that a downward force on the hood (50) results in an outward force on the lower peripheral section (52).

7. The controlled pressure device (10) of claim 1, further comprising a liquid impermeable - air permeable membrane (150) interposed between the reactor (16) and the cosmetic liquid or cream.

8. The controlled pressure device (10) of claim 1, wherein the controlled pressure device (10) is configured to allow an operator to change the size of the enclosed volume (22) while the reactor (16) consumes the selected gas in the enclosed volume (22).

9. The controlled pressure device (10) of claim 1, wherein the reactor (16) is configured having a predetermined scavenging capacity (SC) for the selected gas, wherein the enclosed volume (22) has a determined volume (DV), the controlled pressure device (10) is configured to have a maximum leakage rate (LR) for air entering the enclosed volume (22), and the controlled pressure device (10) is configured to a minimum wear time (MWT) wherein:

$$SC > DV*(\% \text{ of selected gas in air}) + LR*(\% \text{ of selected gas in air})*MWT.$$

10. The controlled pressure device (10) of claim 1, wherein the reactor (16) is one or any combination of a zinc-based chemical pump, an electro-chemical pump, a vacuum-on-demand device, an electrolyzer, a pressure-reducing solid state device, an oxygen absorbing zinc or iron packet, a zinc-air battery, a zinc-air battery component and a getter of zirconium titanium, vanadium iron, lithium, lithium metal, magnesium, calcium, lithium barium combinations.

11. The controlled pressure device (10) of claim 1, wherein the reactor (16) is configured to consume ambient oxygen to heat the cosmetic liquid or cream in the enclosed volume (22).

12. The controlled pressure device (10) of claim 1, wherein a skin contacting element (24) is interposed between the reactor housing element (14) and the tissue around the tissue site (20), said reactor housing element (14) cooperating with the skin contacting element (24) to at least partially define the enclosed volume (22) around the tissue site (20).

13. The controlled pressure device (10) of claim 12, further comprising a skin massaging element positioned in the enclosed volume (22), wherein the skin massaging element is positioned adjacent to the tissue site (20) when a skin contacting side (30) of the skin contacting element (24) is in contact with the subject's skin, wherein the skin massaging element includes an undulated bottom surface that contacts the tissue site (20), wherein the skin massaging element is a membrane (188) including openings through which the cosmetic liquid or cream passes to contact the tissue site (20).

14. A non-therapeutic method of treating a tissue site (20), comprising:

removing a release layer from a reactor housing element (14);

placing the reactor housing element, (14) a reactor, a liquid impermeable - air permeable membrane (150), and a cosmetic liquid or cream over the tissue site (20); and

affixing the reactor housing element (14) with respect to skin around the tissue site (20) to define an at least substantially air-tight enclosed volume (22) around the tissue site (20),

wherein the reactor (16), the liquid impermeable - air permeable membrane (150), and the cosmetic liquid or cream are located in the enclosed volume (22).

15. The method of claim 14, wherein a pad (18) is impregnated or wetted with the cosmetic liquid or cream and is arranged over the tissue site (20).


**Patentansprüche**

1. Vorrichtung (10) mit kontrolliertem Druck zur Behandlung von Gewebe, wobei die Vorrichtung (10) umfasst:

ein Reaktorgehäuseelement (14), welches dazu ausgebildet ist, wenigstens teilweise ein wenigstens im Wesentlichen luftdicht geschlossenes Volumen (22) um eine Gewebestelle (20) herum zu definieren, wenn es räumlich bezüglich der Gewebestelle (20) fixiert ist; und

einen Reaktor (16), welcher in dem geschlossenen Volumen (22) positioniert ist und welcher dazu ausgebildet ist, mit einem in Luft anzutreffenden ausgewählten Gas zu reagieren, wobei der Reaktor (16) das ausgewählte Gas in dem geschlossenen Volumen (22) verbraucht;
**gekennzeichnet durch** eine in dem geschlossenen Volumen (22) angeordnete kosmetische Flüssigkeit oder Creme.

2. Vorrichtung (10) mit kontrolliertem Druck des Anspruchs 1, wobei die kosmetische Flüssigkeit oder Creme wenigstens eine Substanz enthält aus einem Feuchtigkeitsspender, Dimethylaminoethanol (DMAE), Acetylhexapeptid-8, Acetylhexapeptid-3, Retinol, Ubichinon, Dithiolan-3-Pentansäure, Alpha-Hydroxysäure, Alpha-Liponsäure, Salicylsäure, Hydrocortison, topische Botulinum-Creme und Hyaluronsäure.

3. Vorrichtung (10) mit kontrolliertem Druck des Anspruchs 1, ferner umfassend ein Kissen (18), welches mit der kosmetischen Flüssigkeit oder Creme imprägniert ist.

4. Vorrichtung (10) mit kontrolliertem Druck des Anspruchs 1, wobei der Reaktor (16) ein Reaktorsubstrat (80), einen Reduktionswirkstoff, ein Bindemittel und eine Elektrolytlösung umfasst.

5. Vorrichtung (10) mit kontrolliertem Druck des Anspruchs 1, wobei der Reaktor (16) dazu ausgebildet ist, in dem geschlossenen Volumen (22) Gasdruck zu zirkulieren,

wobei der Reaktor (16) eine Mehrzahl von Reaktorelementen (80a, 80b, 80c) umfasst oder
wobei der Reaktor (16) ein erstes Reaktorelement (80a) umfasst, welches dazu ausgebildet ist, zu beginnen, Sauerstoff zu verbrauchen, nachdem es Sauerstoff für eine erste Zeitdauer ausgesetzt ist, und ein zweites Reaktorelement (80b) umfasst, welches dazu ausgebildet ist, zu beginnen, Sauerstoff zu verbrauchen, nachdem es Sauerstoff für eine zweite Zeitdauer ausgesetzt ist, welche größer als die erste Zeitdauer ist, oder
wobei der Reaktor (16) ein erstes Reaktorelement (80a) und ein zweites Reaktorelement (80b) umfasst, wobei das zweite Reaktorelement (80b) dazu ausgebildet ist, mit einer verzögerten Reaktionszeit zu beginnen, Sauerstoff zu verbrauchen, nachdem das erste Reaktorelement (80a) erschöpft ist und nicht weiter Sauerstoff verbraucht.

6. Vorrichtung (10) mit kontrolliertem Druck des Anspruchs 1, wobei das Reaktorgehäuseelement (14) eine Haube (50) und einen unteren Umfangsabschnitt (52) umfasst, welcher die Haube (50) wenigstens teilweise umgibt, wobei das Reaktorgehäuseelement (14) dazu ausgebildet ist, dass eine abwärtsgerichtete Kraft auf die Haube (50) zu einer nach außen gerichteten Kraft auf den unteren Umfangsabschnitt (52) führt.

7. Vorrichtung (10) mit kontrolliertem Druck des Anspruchs 1, ferner umfassend eine flüssigkeitsundurchlässige-luftdurchlässige Membran (150), welche zwischen dem Reaktor (16) und der kosmetischen Flüssigkeit oder Creme angeordnet ist.

8. Vorrichtung (10) mit kontrolliertem Druck des Anspruchs 1, wobei die Vorrichtung (10) mit kontrolliertem Druck (10) dazu ausgebildet ist, einer Bedienperson zu gestatten, die Größe des geschlossenen Volumens (22) zur verändern, während der Reaktor (16) das ausgewählte Gas in dem geschlossenen Volumen (22) verbraucht.

9. Vorrichtung (10) mit kontrolliertem Druck des Anspruchs 1, wobei der Reaktor (16) dazu ausgebildet ist, für das ausgewählte Gas eine vorbestimmte Spülkapazität (SC) aufzuweisen, wobei das geschlossene Volumen (22) ein bestimmtes Volumen (DV) aufweist, wobei die Vorrichtung (10) mit kontrolliertem Druck dazu ausgebildet ist, eine maximale Leckagerate (LR) für in das geschlossene Volumen (22) eintretende Luft aufzuweisen, und wobei die Vorrichtung (10) mit kontrolliertem Druck für eine minimale Tragezeit (MWC) konfiguriert ist, wobei gilt:

$$SC > DV*(\text{\% an ausgewähltem Gas in Luft}) + LR*(\text{\% an ausgewähltem Gas in Luft})*MWT.$$

10. Vorrichtung (10) mit kontrolliertem Druck des Anspruchs 1, wobei der Reaktor (16) eine oder eine beliebige Kombination aus einer zinkbasierten chemischen Pumpe, einer elektrochemischen Pumpe, einer Vorrichtung mit Unterdruckauf-Anforderung, einem Elektrolyseur, einer Vorrichtung mit Druck verringerndem Festkörper, einem Paket aus Sauerstoff absorbierendem Zink oder Eisen, einer Zink-Luft-Batterie, einer Zink-Luft-Batterie-Komponente und einem Fangstoff aus Zirkon-Titan, Vanadium-Eisen, Lithium, Lithiummetall, Magnesium, Calcium, Lithium-Barium-Verbindungen ist.

11. Vorrichtung (10) mit kontrolliertem Druck des Anspruchs 1, wobei der Reaktor (16) dazu ausgebildet ist, Umgebungssauerstoff zu verbrauchen, um die kosmetische Flüssigkeit oder Creme im geschlossenen Volumen (22) zu erwärmen.

12. Vorrichtung (10) mit kontrolliertem Druck des Anspruchs 1, wobei ein Hautkontaktelement (22) zwischen dem Reaktorgehäuseelement (14) und dem Gewebe um die Gewebestelle (20) herum zwischenangeordnet ist, wobei das Reaktorgehäuseelement (14) mit dem Hautkontaktelement (24) zusammenwirkt, um das geschlossene Volumen (22) um die Gewebestelle (20) herum wenigstens teilweise zu definieren.

13. Vorrichtung (10) mit kontrolliertem Druck des Anspruchs 12, ferner umfassend ein Hautmassageelement, welches in dem geschlossenen Volumen (22) angeordnet ist, wobei das Hautmassageelement der Gewebestelle (20) benachbart angeordnet ist, wenn eine Hautkontaktseite (30) des Hautkontaktelements (24) sich in Kontakt mit der Haut eines Subjekts befindet, wobei das Hautmassageelement eine gewellte Bodenoberfläche aufweist, welche die Gewebestelle (20) kontaktiert, wobei das Hautmassageelement eine Membran (188) ist, welche Öffnungen aufweist, durch welche die kosmetische Flüssigkeit oder Creme hindurchtritt, um die Gewebestelle (20) zu kontaktieren.

14. Nicht-therapeutisches Verfahren zur Behandlung einer Gewebestelle (20), umfassend:

Entfernen einer Löselage von einem Reaktorgehäuseelement (14);
Anordnen des Reaktorgehäuseelements (14), eines Reaktors, einer flüssigkeitsundurchlässigen-luftdurchlässigen Membran (150), und einer kosmetischen Flüssigkeit oder Creme über der Gewebestelle (20); und
Befestigen des Reaktorgehäuseelements (14) in Bezug auf die Haut um die Gewebestelle (20) herum, um ein wenigstens im Wesentlichen luftdichtes geschlossenes Volumen (22) um die Gewebestelle (20) herum zu definieren,
wobei der Reaktor (16), die flüssigkeitsundurchlässige-luftdurchlässige Membran (150) und die kosmetische Flüssigkeit oder Creme in dem geschlossenen Volumen (22) angeordnet sind.

15. Verfahren des Anspruchs 14, wobei ein Kissen (18) mit der kosmetischen Flüssigkeit oder Creme imprägniert oder benetzt wird und über der Gewebestelle (20) angeordnet wird.

**Revendications**

1. Dispositif à pression contrôlée (10) pour le traitement de tissus, le dispositif (10) comprenant :

un élément de logement du réacteur (14) configuré pour définir au moins partiellement un volume enfermé au moins essentiellement étanche à l'air (22) autour d'un site de tissu (20) lorsqu'il est fixé dans l'espace par rapport au site de tissu (20); et

un réacteur (16) positionné dans le volume enfermé (22) et configuré pour réagir avec un gaz sélectionné présent dans l'air, dans lequel le réacteur (16) consomme le gaz sélectionné à l'intérieur du volume enfermé (22); **caractérisé par** un liquide ou une crème cosmétique situé dans le volume enfermé (22).

2. Dispositif à pression contrôlée (10) selon la revendication 1, dans lequel le liquide ou la crème cosmétique comprend au moins l'un parmi un hydratant, le diméthylaminoéthanol (DMAE), l'acétyl hexapeptide-8, l'acétyl hexapeptide-3, le rétinol, l'ubiquinone, l'acide dithiolane-3-pentanique, l'acide alpha-hydroxy, l'acide alpha lipoïque, l'acide salicylique, l'hydrocortisone, la crème botulique topique et l'acide hyaluronique.

3. Dispositif à pression contrôlée (10) selon la revendication 1, comprenant en outre une compresse (18) imprégné du liquide ou de la crème cosmétique.

4. Dispositif à pression contrôlée (10) selon la revendication 1, dans lequel le réacteur (16) comprend un substrat de réacteur (80), un agent réducteur, un liant et une solution électrolytique.

5. Dispositif à pression contrôlée (10) selon la revendication 1, dans lequel le réacteur (16) est configuré pour cycler la pression de gaz dans le volume enfermé (22),

dans lequel le réacteur (16) comprend une pluralité d'éléments de réacteur (80a, 80b, 80c), ou
le réacteur (16) comprend un premier élément de réacteur (80a) configuré pour commencer à consommer de l'oxygène après avoir été exposé à l'oxygène pendant une première période de temps et un deuxième élément de réacteur (80b) configuré pour commencer à consommer de l'oxygène après avoir été exposé à l'oxygène pendant une deuxième période de temps, qui est supérieure à la première période de temps, ou
le réacteur (16) comprend un premier élément de réacteur (80a) et un deuxième élément de réacteur (80b), dans lequel le deuxième élément de réacteur (80b) est configuré avec un temps de réaction retardé pour commencer à consommer de l'oxygène après que le premier élément de réacteur (80a) a été épuisé et ne consomme plus d'oxygène.

6. Dispositif à pression contrôlée (10) selon la revendication 1, dans lequel l'élément de logement du réacteur (14) comprend un capot (50) et une section périphérique inférieure (52) entourant au moins partiellement le capot (50), l'élément de logement du réacteur (14) étant configuré de sorte qu'une force vers le bas sur le capot (50) entraîne une force vers l'extérieur sur la section périphérique inférieure (52).

7. Dispositif à pression contrôlée (10) selon la revendication 1, comprenant en outre une membrane imperméable aux liquides - perméable à l'air (150) interposée entre le réacteur (16) et le liquide ou la crème cosmétique.

8. Dispositif à pression contrôlée (10) selon la revendication 1, dans lequel le dispositif à pression contrôlée (10) est configuré pour permettre à un opérateur de modifier la taille du volume enfermé (22) pendant que le réacteur (16) consomme le gaz sélectionné dans le volume enfermé (22).

9. Dispositif de pression contrôlée (10) selon la revendication 1, dans lequel le réacteur (16) est configuré pour avoir une capacité de balayage prédéterminée (SC) pour le gaz sélectionné, dans lequel le volume enfermé (22) a un volume déterminé (DV), le dispositif de pression contrôlée (10) est configuré pour avoir un taux de fuite maximum (LR) pour l'air entrant dans le volume enfermé (22), et le dispositif de pression contrôlée (10) est configuré pour un temps d'usure minimum (MWT) dans lequel :

$$SC > DV*(\% \text{ de gaz sélectionné dans l'air}) + LR*(\% \text{ de gaz sélectionné dans l'air})*MWT.$$

10. Dispositif à pression contrôlée (10) selon la revendication 1, dans lequel le réacteur (16) est une ou toute combinaison d'une pompe chimique à base de zinc, d'une pompe électrochimique, d'un dispositif de vide à la demande, d'un électrolyseur, d'un dispositif à l'état solide réducteur de pression, d'un paquet de zinc ou de fer absorbant l'oxygène, d'une batterie zinc-air, d'un composant de batterie zinc-air et d'un adsorbeur de combinaisons zirconium-titane, vanadium-fer, lithium, lithium métal, magnésium, calcium, lithium-baryum.

11. Dispositif à pression contrôlée (10) selon la revendication 1, dans lequel le réacteur (16) est configuré pour consommer de l'oxygène ambiant pour chauffer le liquide ou la crème cosmétique dans le volume enfermé (22).

12. Dispositif à pression contrôlée (10) selon la revendication 1, dans lequel un élément de contact avec la peau (24) est interposé entre l'élément de logement du réacteur (14) et le tissu autour du site de tissu (20), ledit élément de logement du réacteur (14) coopérant avec l'élément de contact avec la peau (24) pour définir au moins partiellement le volume enfermé (22) autour du site de tissu (20).

13. Dispositif à pression contrôlée (10) selon la revendication 12, comprenant en outre un élément de massage de la peau positionné dans le volume enfermé (22), dans lequel l'élément de massage de la peau est positionné de manière adjacente au site de tissu (20) lorsqu'un côté de contact avec la peau (30) de l'élément de contact avec la peau (24) est en contact avec la peau du sujet, dans lequel l'élément de massage de la peau comprend une surface inférieure ondulée qui est en contact avec le site de tissu (20), dans lequel l'élément de massage de la peau est une membrane (188) comprenant des ouvertures à travers lesquelles le liquide ou la crème cosmétique passe pour être en contact avec le site de tissu (20).

14. Procédé non thérapeutique de traitement d'un site de tissu (20), comprenant:

éliminer une couche de séparation d'un élément de logement du réacteur (14); placer l'élément de logement du réacteur (14), un réacteur, une membrane imperméable aux liquides - perméable à l'air (150), et un liquide ou une crème cosmétique sur le site de tissu (20); et
fixer l'élément de logement du réacteur (14) par rapport à la peau autour du site de tissu (20) pour définir un volume enfermé (22) au moins sensiblement étanche à l'air autour du site de tissu (20),
dans lequel le réacteur (16), la membrane imperméable aux liquides - perméable à l'air (150), et le liquide ou la crème cosmétique sont situés dans le volume enfermé (22).

15. Procédé selon la revendication 14, dans lequel une compresse (18) est imprégné ou mouillé avec le liquide ou la crème cosmétique et est disposé sur le site de tissu (20).

FIG.1

FIG.2

FIG.5

FIG.3

FIG.6

FIG.4

FIG.7

300

REMOVE RELEASE LAYER FROM REACTOR HOUSING ELEMENT

302

PLACE REACTOR HOUSING ELEMENT, REACTOR AND LIQUID IMPERMEABLE-AIR PERMEABLE MEMBRANE OVER TISSUE SITE

304

AFFIX REACTOR HOUSING ELEMENT WITH RESPECT TO SKIN

## FIG.8

310

PLACE COSMETIC LIQUID OR CREAM ON TISSUE SITE

312

PLACE PAD ON TISSUE SITE

314

REMOVE RELEASE LAYER FROM SKIN CONTACTING ELEMENT

316

PLACE SKIN CONTACTING ELEMENT OVER OR AROUND TISSUE SITE

318

AFFIX REACTOR HOUSING ELEMENT TO SKIN CONTACTING ELEMENT

320

AFFIX SKIN CONTACTING ELEMENT TO SKIN AROUND TISSUE SITE

## FIG.9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050070835 A1
- US 20080183119 A1
- US 20060235358 A1
- US 20140109890 A1
- WO 2015054040 A1